# EUROPÄISCHE PATENTSCHRIFT

(11) **EP 2 701 637 B1**
(45) Veröffentlichungstag und Bekanntmachung des Hinweises auf die Patenterteilung: **20.07.2016**
(21) Anmeldenummer: 12724557.9
(22) Anmeldetag: 13.04.2012
(51) Int. Cl.: A61F 2/44, A61F 2/46

(54) **WIRBELSÄULENIMPLANTAT, WERKZEUG HIERFÜR UND VERFAHREN ZUR DISTRAKTION DES WIRBELSÄULENIMPLANTATES**
SPINAL IMPLANT, TOOL THEREFOR AND METHOD FOR DISTRACTING THE SPINAL IMPLANT
IMPLANT VERTÉBRAL, OUTIL POUR CET IMPLANT ET PROCÉDÉ POUR LA DISTRACTION DE L'IMPLANT VERTÉBRAL

(30) Priorität: 26.04.2011 DE 102011018692
(43) Veröffentlichungstag der Anmeldung: 05.03.2014
(73) Patentinhaber: Metz-Stavenhagen, Peter, 34537 Bad Wildungen (DE)
(72) Erfinder: Metz-Stavenhagen, Peter, 34537 Bad Wildungen (DE)
(74) Vertreter: Walther, Walther & Hinz GbR
(86) Internationale Anmeldenummer: PCT/DE2012/000392
(87) Internationale Veröffentlichungsnummer: WO 2012/146231

(56) Entgegenhaltungen:
- WO-A1-2011/134457
- US-A1- 2002 082 696
- US-A1- 2006 004 447
- US-A1- 2006 241 770
- US-A1- 2008 243 254

## Beschreibung

Die vorliegende Erfindung betrifft ein Wirbelsäulenimplantat gemäß dem Oberbegriff des Anspruches 1, sowie ein Werkzeug hierfür gemäß dem Oberbegriff des Anspruches 4 oder 5 und ein Verfahren zum Distrahieren des Wirbelsäulenimplantates gemäß dem Oberbegriff des Anspruches 8 oder des Anspruches 9.

Aus der US 7,029,498 B2 ist ein aus zwei U-förmigen Teilen zusammengesetztes, distrahierbares Wirbelsäulenimplantat bekannt, bei dem die beiden Teile teleskopartig gegeneinander axial verschiebbar gehalten sind. An den freien Stegen des U-förmigen Außenteiles ist eine Transportaufnahme ausgebildet, in die eine Faßzange einsetzbar ist. Mit dieser Faßzange kann der behandelnde Arzt das Wirbelsäulenimplantat ergreifen und zum gewünschten Ort transportieren.

Nachdem das Wirbelsäulenimplantat platziert ist wird die Faßzange wieder entfernt. Zum Distrahieren des Wirbelsäulenimplantates auf die gewünschte Größe wird als nächstes ein länglicher Führungsstab an der Öffnung des U-förmigen Teiles vorbei ins Innere des Wirbelsäulenimplantates geführt und in ein am Außenteil vorhandenes Gewinde eingeschraubt, bevor ein hohles Verzahnungsinstrument über den Führungsstab geschoben wird. Dabei wird das Verzahnungsinstrument soweit in das Wirbelsäulenimplantat eingeschoben, bis an dem Verzahnungsinstrument vorhandene Außenzähne in entsprechend ausgebildete Zähne am Innenteil des Wirbelsäulenimplantates eingreifen. Dreht man nun das Verzahnungsinstrument um seine Längsachse, so wird das Innenteil des Wirbelsäulenimplantates gegenüber dem Außenteil verschoben.

Dieser ganze Vorgang ist sehr schwierig und erfordert ein hohes Maß an Fingerfertigkeit des operierenden Arztes. Weil das Verzahnungsinstrument nur sehr lose auf dem Führungsstab sitzt kann es während dem Distrahieren vorkommen, dass es unbeabsichtigterweise aus dem Eingriff mit den Zähnen herausrutscht, so dass es neu eingeführt werden muss.

Aus der US 2006/0241770 A1 ist ein Wirbelsäulenimplantat bekannt, welches einen Außen- und einen axial darin verschieblich gehaltenen Innenkorpus aufweist, wobei auf einer Stirnseite des Außenkorpus ein drehbarer Drehring angeordnet ist, an dessen Innenseite ein Gewinde ausgebildet ist, wobei dieses Gewinde in ein Außengewinde des Innenkorpus eingreift. Zum axialen Verstellen des Innenkorpus über den Außenkorpus wird ein entsprechendes Werkzeug mit einem langen Stift durch eine erste Öffnung im Außen- und Innenkorpus hindurch bis zu einer Hebelaufnahme auf der Innenseite des Innenkorpus eingesteckt, wobei ein am Werkzeug ausgebildeter Zahnkranz in einen entsprechenden Zahnkranz am Drehring eingreift. Wird nun das Werkzeug in seiner Längsachse rotiert, so bewegt sich der Drehring um die Hochachse des Wirbelsäulenimplantates. Über das Gewinde am Drehring wird der Innenkorpus entsprechend der Steigung des Gewindes axial bewegt. Ein solches Wirbelsäulenimplantat setzt sich aus vielen Einzelteilen zusammen, was zu einer kostenintensiven Herstellung führt. Die vielen gegeneinander beweglichen Einzelteile bewirken aber auch eine hohe Reibung und die Gefahr eines Verkantens. Darüber hinaus muss der behandelnde Arzt eine hohe Fingerfertigkeit aufweisen, um das Werkzeug mit seinem Stift zunächst in die erste Öffnung im Innen- und im Außenkorpus hindurch bis in die auf der gegenüberliegenden Seite Hebelaufnahme zu führen, wobei der Zahnkranz und der Stift zeitgleich in die Hebelaufnahme bzw. in den Zahnkranz am Drehring eingreifen muss, um das Werkzeug wirksam benutzen zu können.

Aus der nachveröffentlichten WO 2011/134457 A1 ist ein Wirbelsäulenimplantat mit einem Außenkorpus und mit einem axial verschieblich angeordneten Innenkorpus bekannt, wobei am Außenkorpus eine Gewindeaufnahme zur Aufnahme eines entsprechenden Werkzeuges vorgesehen ist. Dabei wird das Werkzeug fest in den Außenkorpus eingeschraubt und greift mit einem Stift in.ein Langloch am Innenkorpus ein. Der Stift besitzt einen umlaufenden Zahnkranz, welcher in entsprechende am Langloch eingelassene Zähne eingreift. Beim Rotieren des Stiftes wird somit der Innenkorpus axial bewegt.

Davon ausgehend liegt der vorliegenden Erfindung die Aufgabe zu Grunde, ein Wirbelsäulenimplantat und ein Werkzeug hierfür der eingangs genannten Art zu schaffen, so dass das Wirbelsäulenimplantat in einfacher Weise und präzise distrahiert werden kann.

Als technische Lösung dieser Aufgabe wird erfindungsgemäß ein Wirbelsäulenimplantat der eingangs genannten Art mit den Merkmalen des Anspruches 1, ein Werkzeug mit den Merkmalen des Anspruches 3, sowie ein Verfahren zum Distrahieren des Wirbelsäulenimplantates gemäß den Merkmalen des Anspruches 6 vorgeschlagen. Vorteilhafte Weiterbildungen dieses Wirbelsäulenimplantates, dieses Werkzeuges und dieses Verfahrens sind den jeweiligen Unteransprüchen zu entnehmen.

Ein nach dieser technischen Lehre ausgebildetes Wirbelsäulenimplantat hat den Vorteil, dass aufgrund der gegenläufigen Bewegung von Innen- und Außenkorpus ein schnelles distrahieren erreicht wird, weil mit einer Handbewegung gleich beide Korpi bewegt werden.

Ein weiterer Vorteil besteht darin, dass durch den vergleichsweise großen Hub eine präzise Einstellung des Wirbelsäulenimplantates erreicht wird. Weiter auf Seite 3 der ursprünglichen Beschreibung Noch ein weiterer Vorteil besteht darin, dass es Aufgrund des formschlüssigen Eingriffes des Werkzeuges in die Wandungszähne auch möglich, den Innenkorpus, bzw. den Außenkorpus, mit geringem Kraftaufwand zu distrahieren, selbst wenn dieser bereits implantiert ist und sogar, wenn dieser unter Belastung steht.

Die zweiteilige Ausführung des Werkzeuges hat den Vorteil, dass in sehr präziser Form entweder der Innenkorpus und/oder der Außenkorpus bewegt werden kann. Dies gibt dem behandelnden Arzt die Möglichkeit, je nach individueller Situation nur den Innenkorpus oder nur den Außenkorpus zu bewegen.

In einer bevorzugten Ausführungsform sind am Innen- und am Außenkorpus Führungsmittel vorgesehen, die ein Verdrehen des Innenkorpus gegenüber dem Außenkorpus verhindern und gleichzeitig eine axiale Führung gewährleisten. Dies hat den Vorteil, dass beim Distrahieren etwaig auftretende Umfangskräfte nicht zum Verdrehen des Innenkorpus führen.

Es hat sich als vorteilhaft erwiesen, diese Führungsmittel nach dem Nut-Feder-Prinzip auszugestalten. Dabei ist beispielsweise im Innenkorpus eine koaxial angeordnete Nut ausgebildet, in die einen korrespondierend hierzu ausgebildeten Vorsprung an einer Innenwandung des Außenkorpus eingreift. Durch diese Nut-Feder Konstruktion erfolgt die Führung des Innenkorpus über eine gewisse Länge des Wirbelsäulenimplantates, so dass ein Verkanten zuverlässig verhindert wird.

Weitere Vorteile des erfindungsgemäßen Wirbelsäulenimplantates, des erfindungsgemäßen Werkzeuges und des erfindungsgemäßen Verfahrens ergeben sich aus der beigefügten Zeichnung und den nachstehend beschriebenen Ausführungsformen. Ebenso können die vorstehend genannten und die noch weiter ausgeführten Merkmale erfindungsgemäß jeweils einzeln oder in beliebigen Kombinationen miteinander verwendet werden. Die erwähnten Ausführungsformen sind nicht als abschließende Aufzählung zu verstehen, sondern haben vielmehr beispielhaften Charakter. Es zeigen:
- Fig. 1: eine perspektivische Darstellung eines erfindungsgemäßen Wirbelsäulenimplantates und einer ersten Ausführungsform eines erfindungsgemäßen Werkzeuges;
- Fig. 2: eine geschnitten dargestellte Draufsicht auf das Wirbelsäulenimplantat und das Werkzeug gemäß Fig. 1, geschnitten entlang Linie II - II in Fig. 1;
- Fig. 3: eine geschnitten dargestellte Seitenansicht des Wirbelsäulenimplantates gemäß Fig. 1, geschnitten entlang Linie III - III in Fig. 2;
- Fig. 4: eine perspektivische Darstellung des Wirbelsäulenimplantates gemäß Fig. 1 und einer zweiten Ausführungsform eines erfindungsgemäßen Werkzeuges;
- Fig. 5: eine geschnitten dargestellte Draufsicht auf das Wirbelsäulenimplantat gemäß Fig. 1 und des Werkzeuges gemäß Fig. 4;
- Fig. 6: eine geschnitten dargestellte Seitenansicht des Wirbelsäulenimplantates gemäß Fig. 1, geschnitten entlang Linie VI - VI in Fig. 5.

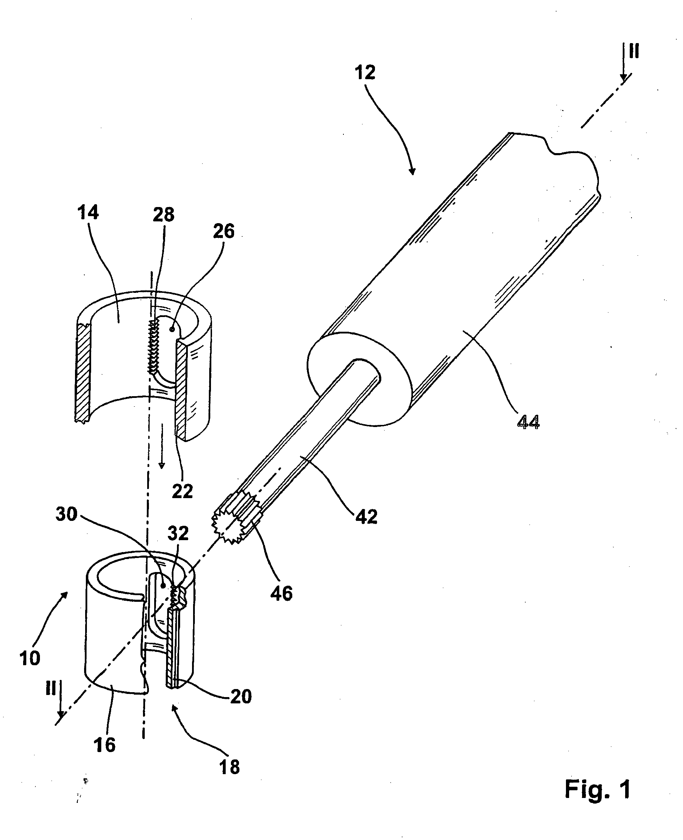

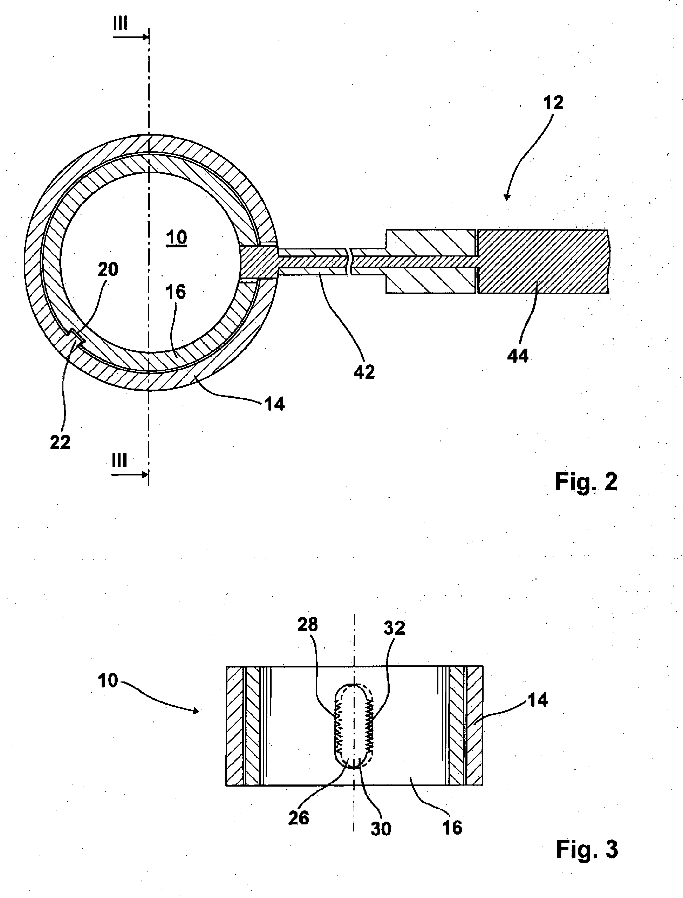

In den Fig. 1 bis 3 ist ein erfindungsgemäßes Wirbelsäulenimplantat 10 und eine erste Ausführungsform eines erfindungsgemäßen Werkzeuges 12 dargestellt.

Das Wirbelsäulenimplantat umfasst einen Außenkorpus 14 und einen axial verschieblich darin gehaltenen Innenkorpus 16, die beide in der hier dargestellten Ausführungsform zylindrisch ausgeführt sind. An den beiden Korpi 14, 16 sind Führungsmittel 18 ausgebildet, die die axiale Bewegung der beiden Korpi 14, 16 führt und ein Verdrehen der Korpi 14, 16 gegeneinander sperrt. Das Führungsmittel 18 umfasst eine an einer Außenseite des Innenkorpus 16 eingelassene, koaxial ausgerichtete Nut 20 und einen korrespondierend zur Nut 20 an einer Innenseite des Außenkorpus 14 ausgebildeten Vorsprung 22.

Im Innenkorpus 16 ist eine als koaxial ausgerichtetes Langloch ausgebildete erste Hebelaufnahme 26 ausgebildet, an dessen vertikaler Flanke eine Anzahl von Wandungszähnen 28 ausgebildet sind. Im Außenkorpus 14 ist eine zweite, ebenfalls als koaxial ausgerichtetes Langloch ausgebildete Hebelaufnahme 30 vorgesehen, an dessen vertikaler Flanke eine Anzahl von Wandungszähnen 32 ausgebildet sind. Die Wandungszähne 28, 32 der ersten und zweiten Hebelaufnahme 26, 30 sollten an gegenüberliegen Flanken der Hebelaufnahme 26, 30 vorgesehen sein.

Die Nut 20, der Vorsprung 22, der Innenkorpus 16 und der Außenkorpus 14 sind so angeordnet, dass die erste als Langloch ausgebildete Hebelaufnahme 26 mit der zweiten als Langloch ausgebildeten Hebelaufnahme 30 zumindest teilweise fluchtet, so dass ein Werkzeug 12 durch die zweite Hebelaufnahme 30 hindurch bis in die erste Hebelaufnahme 26 gesteckt werden kann.

Die erste Ausführungsform eines erfindungsgemäßen Werkzeugs 12 setzt sich zusammen aus einem stabähnlichen Hebeldreher 42 und einem daran angebrachten Griff 44. Am distalen Ende des Hebeldrehers 42 ist eine Anzahl radial abstehender Zähne 46 vorgesehen, die koaxial zur Längsachse des Hebeldrehers 42 an dessen Oberfläche ausgebildet sind. Die Zähne 46 sind so eng angeordnet, dass sie einen Zahnkranz bilden. Auch ist jeder Zahn 46 so lang ausgeführt, dass er gleichzeitig in einen korrespondierenden Wandungszahn 28 einer ersten Hebelaufnahme 26 und in einen korrespondierenden Wandungszahn 32 einer zweiten Hebelaufnahme 30 des Wirbelsäulenimplantates 10 eingreifen kann.

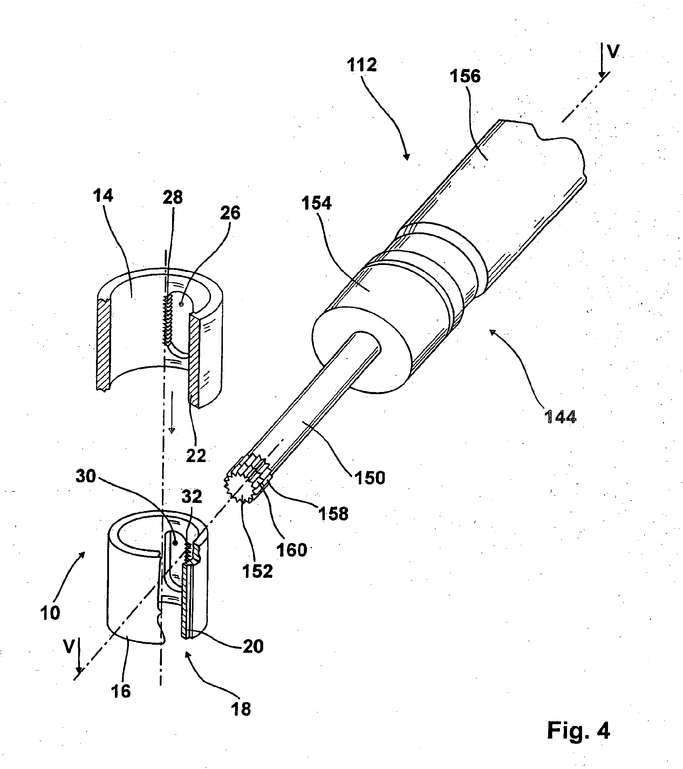

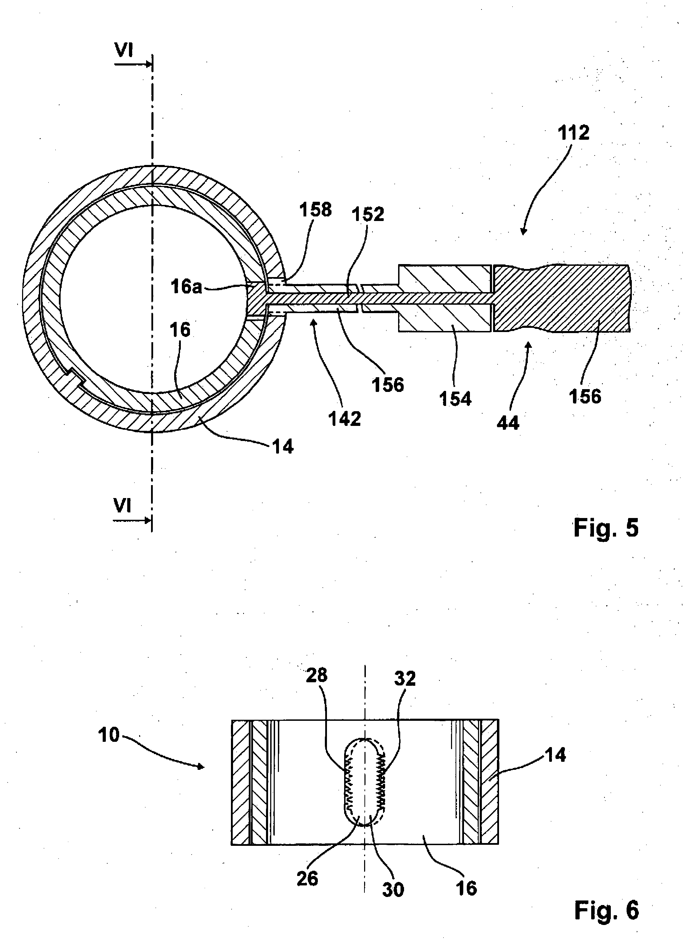

In den Fig. 4 bis 6 ist eine zweite Ausführungsform eines erfindungsgemäßen Werkzeuges 112 dargestellt, welches sich von der in den Fig. 1 bis 3 dargestellten ersten Ausführungsform lediglich dadurch unterscheidet, dass der Hebeldreher 142 des Werkzeugs gemäß Fig. 4 bis 6 zweigeteilt ausge-führt ist und über einen Innendreher 150 und einen Außendreher 152 verfügt. Auch der Griff 144 ist zweigeteilt und verfügt über einen Innengriff 154 und einen Außengriff 156.

Der Innendreher 150 ist mit dem Innengriff 154 wirkverbunden und der Außengriff 156 ist mit dem Außendreher 152 wirkverbunden, so dass der Innendreher 150 über den Innengriff 154 und der Außendreher 152 über den Außengriff 154 betätigt werden kann. Dabei können beide unabhängig voneinander benutzt werden. Hierzu ist der Innendreher 150 und der Innengriff 154 hohl ausgeführt, so dass der Außendreher 152 in diesem Hohlraum gehalten wird.

Am distalen Ende des Innendrehers 150 und am distalen Ende des Außendrehers 152 sind Innenzähne 158 und Außenzähne 160 radial abstehend angeordnet, die beide die gleiche Länge aufweisen.

Während eines chirurgischen Eingriffes bestimmt der behandelnde Arzt die Größe des einzusetzenden Wirbelsäulenimplantates 10. Danach wird das Wirbelsaulenimplantat an die gewünschte Stelle im Körper eingesetzt. Zum Distrahieren wird nun das Werkzeug 12 gemäß der ersten Ausführungsform (Fig. 1 bis 3) derart in das Wirbelsäulenimplantat eingesetzt, dass die Zähne 46 sowohl in die erste Hebelaufnahme 26, als auch in die zweite Hebelaufnahme 30 hineinreichen und dort mit den Wandungszähnen 28, 32 in Eingriff kommen. Nun wird der behandelnde Arzt den Griff 44 des Werkzeuges 12 um seine Längsachse verdrehen. Dabei bewirkt der Hebeldreher 42 gleichzeitig ein Verschieben des Innenkorpus 16 in die eine und ein Verschieben des Außenkorpus 14 in die andere Richtung. Weil mit einer einzigen Handbewegung beide Korpi 14 und 16 bewegt werden, kann die Distraktion sehr schnell und sehr präzise ausgeführt werden.

Bei Verwendung des Werkzeuges 112 gemäß Fig. 4 bis 6 greifen die Außenzähne 160 in die erste Hebelaufnahme 26 im Innenkorpus 16 und die Innenzähne 158 in die zweite Hebelaufnahme 30 im Außenkorpus 14 ein. Nun kann durch Verdrehen des Innengriffes 154 der Außenkorpus 14 oder durch Verdrehen des Außengriffes 156 der Innenkorpus 16 axial bewegt werden.

## Patentansprüche

1. Wirbelsäulenimplantat mit einem Außenkorpus (14) und mit einem axial verschieblich darin gehaltenen Innenkorpus (16),
wobei am Außenkorpus (14) eine erste Hebelaufnahme (26) und am Innenkorpus (16) eine zweite Hebelaufnahme (30) vorgesehen ist,
**dadurch gekennzeichnet,**
**dass** beide Hebelaufnahmen (26, 30) als Langloch ausgebildet sind, und wobei beide Hebelaufnahmen (26, 30) miteinander fluchtend angeordnet sind, dass an einer Längsseite der ersten und der zweiten Hebelaufnahme (26, 30) eine Anzahl in die Hebelaufnahme (26, 30) hineinragender Wandungszähne (28, 32) ausgebildet sind, dass die Wandungszähne (28, 32) in ihrer jeweiligen Hebelaufnahme (26, 30) an sich gegenüberliegenden Seiten angeordnet sind, und
**dass** am Innen- (16) und am Außenkorpus (14) Führungsmittel (18) vorgesehen sind, die ein axiales Verschieben des Innenkorpus (16) gegenüber dem Außenkorpus (14) erlauben, die aber ein Verdrehen des Innenkorpus (16) gegenüber dem Außehkorpus (14) verhindern.

2. Wirbelsäulenimplantat nach Anspruch 1,
**dadurch gekennzeichnet,**
**dass** die Führungsmittel (18) nach dem Nut-Feder-Prinzip ausgebildet sind.

3. Werkzeug für ein insbesondere nach einem der Ansprüche 1 bis 2 ausgebildetes Wirbelsäulenimplantat, mit einem stabähnlichen Hebeldreher (142) und einem daran angebrachten Griff (144),
**dadurch gekennzeichnet,**
**dass** der Hebeldreher (142) zweiteilig ausgeführt ist, wobei der Hebeldreher (142) einen Innen- (150) und einen Außendreher (152) umfasst und dass der Innendreher (150) an seinem distalen Ende einen Innenzahn (158) und der Außendreher (152) an seinem distalen Ende einen Außenzahn (160) aufweist, so dass der Innendreher (150) unabhängig vom Außendreher (152) benutzt werden kann.

4. Werkzeug nach Anspruch 3,
**dadurch gekennzeichnet,**
**dass** der Griff (144) zweiteilig ausgeführt ist, wobei der Griff (144) einen Innengriff (154) und einen Außengriff (156) umfasst und dass der Innendreher (150) mit dem Innengriff (154) und der Außendreher (152) mit dem Außengriff (156) wirkverbunden ist.

5. Werkzeug nach einem der Ansprüche 3 oder 4,
**dadurch gekennzeichnet,**
**dass** der Innenzahn (158) und der Außenzahn (160) die gleiche Länge aufweisen.

6. Verfahren zum Distrahieren eines nach einem der Ansprüche 1 bis 2 ausgebildeten Wirbelsäulenimplantates, mittels eines nach einem der Ansprüche 3 bis 5 ausgebildeten Werkzeuges,
**dadurch gekennzeichnet,**
**dass** zunächst das Werkzeug (112) mit seinem Hebeldreher (142) in die beiden Hebelaufnahmen (26, 30) am Innen- (16) und am Außenkorpus (14) des Wirbelsäulenimplantates (10) derart eingeführt wird, dass der Innendreher (150) mit seinen Innenzähnen (158) mit den Wandungszähnen (32) der Hebelaufnahme (30) des Außenkorpus (14) in Eingriff ist und dass der Außendreher (152) mit seinen Außenzähnen (160) mit den Wandungszähnen (28, 32) der Hebelaufnahme (26, 30) des Innenkorpus (14) in Eingriff ist, bevor das Wirbelsäulenimplantat (10) durch Verdrehen entweder des Innendrehers (150) oder des Außendrehers (152) oder beider distrahiert wird.

7. Verfahren nach Anspruch 6,
**dadurch gekennzeichnet,**
**dass** der Innendreher (150) durch Verdrehen des Innengrififes (154) um seine Längsachse betätigt wird und/oder dass der Außendreher (152) durch Verdrehen des Außengriffes (156) um seine Längsachse betätigt wird.

## Claims

1. A spinal implant with an external body (14) and with an axially shiftable internal body (16) held in the external body (14), with a first lever receptacle (26) being provided on the external body (14) and a second lever receptacle (30) being provided on the internal body (16),
**characterized in**
**that** both lever receptacles (26, 30) are formed as an elongated hole, and with both lever receptacles (26, 30) being arranged in alignment with each other, that on a longitudinal side of the first and second lever receptacle (26, 30) a number of wall teeth (28, 32) extending into the lever receptacle (26, 30) are formed, that the wall teeth (28, 32) in their respective lever receptacle (26, 30) are arranged on opposite sides, and that guiding means (18) are provided on the internal (16) and external body (14) permitting an axial shifting of the internal body (16) relative to the external body (14) but preventing twisting of the internal body (16) relative to the external body (14).

2. The spinal implant according to claim 1,
**characterized in**
**that** the guiding means (18) are configured according to the tongue-and-groove principle.

3. A tool for a spinal implant formed in particular according to anyone of claims 1 to 2 comprising a rod-like lever-rotator (142) and a handle (144) attached to it,
**characterized in**
**that** the lever-rotator (142) is designed in a bipartite manner, with the lever-rotator (142) comprising an internal rotator (150) and an external rotator (152) and that the internal rotator (150) comprises an internal tooth (158) at its distal end and the external rotator (152) comprises an external tooth (160) at its distal end so that the internal rotator (150) can be used independently of the external rotator (152).

4. The tool according to claim 3,
**characterized in**
the handle (144) is designed in a bipartite manner with the handle (144) comprising an internal handle (154) and an external handle (156) and that the internal rotator (150) is operatively connected with the internal handle 154 and the external rotator (152) is operatively connected with the external handle (156).

5. The tool according to claim 3 or 4,
**characterized in**
the internal tooth (158) and the external tooth (160) have the same length.

6. A method for distracting a spinal implant formed according to anyone of claims 1 to 2 by means of a tool formed according to any one claims 3 to 5,
**characterized in**
**that** at first the tool (112) with its lever-rotator (142) is inserted into the two lever receptacles (26, 30) on the internal body (16) and on the external body (14) of the spinal implant (10) in such a way that the internal rotator (150) with its internal teeth (158) engages into the wall teeth (32) of the lever receptacle 30 of the external body (14), and that the external rotator (152) with its external teeth (160) engages into the wall teeth (28, 32) of the lever receptacle (26, 30) of the internal body (14), before the spinal implant (10) is distracted by turning either the internal rotator (150) or the external rotator (152) or both.

7. The method according to claim 6,
**characterized in**
**that** the internal rotator (150) is actuated by turning the internal handle (154) around its longitudinal axis and/or that the external rotator (152) is actuated by turning the external handle (156) around its longitudinal axis.

## Revendications

1. Implant vertébral avec un corps externe (14) et un corps interne (16) maintenu dans le corps externe (14) axialement coulissant,
un premier logement de levier (26) étant prévu au corps externe (14) et un deuxième logement de levier (30) étant prévu au corps interne (16),
**caractérisé en ce**
**que** les deux logements de levier (26, 30) sont formés en tant que trou oblong, et les deux logements de levier (26, 30) étant disposés en alignement l'un par rapport à l'autre, que sur un côté longitudinal du premier et du deuxième logement de levier (26, 30) un nombre de dents de paroi (28, 32) s'étendant dans le logement de levier (26, 30) est formé, que les dents de paroi (28, 32) dans leur logement de levier (26, 30) respectif sont disposés sur des côtés opposés l'un à l'autre, et que des moyens de guidage (18) sont prévus au corps interne (16) et au corps externe (14) permettant un déplacement axial du corps interne (16) par rapport au corps externe (14) mais évitant une torsion du corps interne (16) par rapport au corps externe (14).

2. Implant vertébral selon la revendication 1,
**caractérisé en ce**
**que** les moyens de guidage (18) sont formé selon le principe rainure-languette.

3. Outil pour un implant vertébral surtout formé selon l'une quelconque des revendications 1 à 2, avec un organe de rotation de levier en forme de tige (142) et une poignée (144) qui y est fixée,
**caractérisé en ce**
**que** l'organe de rotation de levier (142) est réalisé en deux parties, l'organe de rotation de levier (142) comportant un organe de rotation interne (150) et un organe de rotation externe (152) et que l'organe de rotation interne (150) à son extrémité distale comprend un dent interne (158) et que l'organe de rotation externe (152) à son extrémité distale comprend un dent externe (160) de manière que l'organe de rotation interne (150) peut être utilisé indépendamment de l'organe de rotation externe (152).

4. Outil selon la revendication 3,
**caractérisé en ce**
**que** la poignée (144) est réalisée en deux parties, la poignée (144) comportant une poignée interne (154) et une poignée externe (156) et que l'organe de rotation interne (150) est opérativement connecté avec la poignée interne (154) et l'organe de rotation externe (152) est opérativement connecté avec la poignée externe (156).

5. Outil selon l'une quelconque des revendications 3 ou 4,
**caractérisé en ce**
**que** le dent interne (158) et le dent externe (160) ont la même longueur.

6. Procédé pour la distraction d'un implant vertébral formé selon l'une quelconque des revendications 1 à 2, au moyen d'un outil selon l'une quelconque des revendications 3 à 5,
**caractérisé en ce**
**que** d'abord l'outil (112) avec son organe de rotation de levier (142) est introduit dans les deux logements de levier (26, 30) au corps interne (16) et au corps externe (14) de l'implant vertébral (10) de sorte que le l'organe de rotation interne (150) avec ses dents internes (158) est engrené dans les dents de paroi (32) du logement de levier (30) du corps externe (14), et que l'organe de rotation externe (152) avec ses dents externes (160) est engrené dans les dents de paroi (32) du logement de levier (26, 30) du corps interne (14) avant que l'implant vertébral (10) par rotation soit de l'organe de rotation interne (150) soit de l'organe de rotation externe (152) ou les deux est distracté.

7. Procédé selon la revendication 6,
**caractérisé en ce**
**que** l'organe de rotation interne (150) est actionné en tournant la poignée interne (154) autour de son axe longitudinal et/ou que l'organe de rotation externe (152) est actionné en tournant la poignée externe (156) autour de son axe longitudinal.
